# EUROPEAN PATENT APPLICATION

(11) **EP 1 752 537 A1**
(43) Date of publication of application: **14.02.2007**
(21) Application number: 05743243.7
(22) Date of filing: 27.05.2005
(51) Int. Cl.: C12N 15/11, C12N 9/88

(54) **9-FATTY ACID HYDROPEROXIDE LYASE GENES**

(30) Priority: 31.05.2004 JP 2004162138; 01.11.2004 JP 2004318233; 20.12.2004 JP 2004368306
(71) Applicant: Sapporo Breweries Limited, Tokyo 150-8522 (JP)
(72) Inventor: KURODA, Hisao, c/o Sapporo Breweries Limited, Yaizu-shi, Shizuoka 4250013 (JP); KANEDA, Hirotaka, c/o Sapporo Breweries Limited, Yaizu-shi, Shizuoka 4250013 (JP); OOSHIMA, Toshiyuki, c/o Sapporo Breweries Limited, Yaizu-sh, Shizuoka 4250013 (JP)
(74) Representative: Vossius & Partner
(86) International application number: PCT/JP2005/009757
(87) International publication number: WO 2005/116211

(57) **Abstract**

A 9-fatty acid hydroperoxide lyase gene consisting of the nucleotide sequence as set forth in SEQ ID No: 1, and a 9-fatty acid hydroperoxide lyase gene consisting of the nucleotide sequence as set forth in SEQ ID No: 5.

SEQUENCE LISTING

<110>  Sapporo Breweries Ltd.

<120>  9-FATTY ACID HYDROPEROXIDE LYASE GENES

<130>  FP05-0209-00

<150>  JP 2004-162138
<151>  2004-05-31

<150>  JP 2004-318233
<151>  2004-11-01

<150>  JP 2004-368306
<151>  2004-12-20

<160>  8

<170>  PatentIn version 3.1

<210>  1
<211>  1533
<212>  DNA
<213>  Oryza sativa

<220>
<221>  CDS
<222>  (1)..(1533)
<223>

<400>  1
atg gcg ccg ccg cga gcc aac tcc ggc gac ggt aac gac ggc gcc gtc      48
Met Ala Pro Pro Arg Ala Asn Ser Gly Asp Gly Asn Asp Gly Ala Val
1               5                   10                  15

gga ggg cag agc aag ctc tcg ccg tcg ggc ctg ctg ata cgc gag att      96
Gly Gly Gln Ser Lys Leu Ser Pro Ser Gly Leu Leu Ile Arg Glu Ile
            20                  25                  30

ccg ggc ggc tac ggc gtg ccc ttc ctc tcg ccg ctg cgc gac cgc ctc      144
Pro Gly Gly Tyr Gly Val Pro Phe Leu Ser Pro Leu Arg Asp Arg Leu
        35                  40                  45

gac tac tat tac ttc cag ggc gcc gac gag ttc ttc cgc tca cgc gtc      192
Asp Tyr Tyr Tyr Phe Gln Gly Ala Asp Glu Phe Phe Arg Ser Arg Val
    50                  55                  60

gcc cgc cac ggc ggc gcc acc gtg ctc cgc gtc aac atg ccg ccc ggc      240
Ala Arg His Gly Gly Ala Thr Val Leu Arg Val Asn Met Pro Pro Gly
65                  70                  75                  80

ccc ttc ctc gcc ggc gac ccc cgc gtc gtc gcc ctc ctc gac gcg cgc      288
Pro Phe Leu Ala Gly Asp Pro Arg Val Val Ala Leu Leu Asp Ala Arg
                85                  90                  95

agc ttc cgc gtc ctc ctc gac gac tcc atg gtg gac aag gcc gac acg      336
Ser Phe Arg Val Leu Leu Asp Asp Ser Met Val Asp Lys Ala Asp Thr
            100                 105                 110

ctc gac ggc acc ttc atg ccg tcg ctc gcg ctc ttc ggc ggc cac cgc      384
Leu Asp Gly Thr Phe Met Pro Ser Leu Ala Leu Phe Gly Gly His Arg
        115                 120                 125

ccg ctc gcc ttc ctc gac gcc gcc gac cct cgc cac gcc aag atc aag      432
Pro Leu Ala Phe Leu Asp Ala Ala Asp Pro Arg His Ala Lys Ile Lys
    130                 135                 140

cgc gtc gtg atg tcg ctc gcc gcg gcg agg atg cac cac gtc gcg ccg      480
Arg Val Val Met Ser Leu Ala Ala Ala Arg Met His His Val Ala Pro
145                 150                 155                 160

gcg ttc cgc gcc gcc ttc gcc gcc atg ttc gac gag gtc gac gcc ggc      528
Ala Phe Arg Ala Ala Phe Ala Ala Met Phe Asp Glu Val Asp Ala Gly
                165                 170                 175

ctc gtc gcc ggc ggc ccc gtc gag ttc aac aag ctc aac atg cgg tac 576
Leu Val Ala Gly Gly Pro Val Glu Phe Asn Lys Leu Asn Met Arg Tyr
            180                 185                 190

atg ctc gac ttc acc tgc gcc gcg ctg ttc ggc ggc gcg ccg ccg agc 624
Met Leu Asp Phe Thr Cys Ala Ala Leu Phe Gly Gly Ala Pro Pro Ser
        195                 200                 205

aag gcc atg ggc gac gct gcc gtg acg aag gcg gtg aag tgg ctc atc 672
Lys Ala Met Gly Asp Ala Ala Val Thr Lys Ala Val Lys Trp Leu Ile
    210                 215                 220

ttc cag ctt cac ccg ctc gcc agc aag gtc gtc aag ccg tgg ccg ctg 720
Phe Gln Leu His Pro Leu Ala Ser Lys Val Val Lys Pro Trp Pro Leu
225                 230                 235                 240

gag gac ctc ctc ctc cac acc ttc cgc ctg ccg ccg ttc ctg gtg cgc 768
Glu Asp Leu Leu Leu His Thr Phe Arg Leu Pro Pro Phe Leu Val Arg
                245                 250                 255

cgc gag tac ggc gag atc acg gcg tac ttc gcc gcc gcc gcc gcg gcc 816
Arg Glu Tyr Gly Glu Ile Thr Ala Tyr Phe Ala Ala Ala Ala Ala Ala
            260                 265                 270

atc ctc gac gac gcc gag aag aac cac ccg gga atc ccg cgc gac gag 864
Ile Leu Asp Asp Ala Glu Lys Asn His Pro Gly Ile Pro Arg Asp Glu
        275                 280                 285

ctc ctc cac aac ctc gtg ttc gtc gcc gtc ttc aac gcc tac ggc ggc 912
Leu Leu His Asn Leu Val Phe Val Ala Val Phe Asn Ala Tyr Gly Gly
    290                 295                 300

ttc aag atc ttc ctg cca cac atc gtc aag tgg ctc gcc cgc gcc ggc 960
Phe Lys Ile Phe Leu Pro His Ile Val Lys Trp Leu Ala Arg Ala Gly
305                 310                 315                 320

ccg gag ctc cac gcc aag cta gcc tcc gag gtc cgc gcc gcc gcg ccc 1008
Pro Glu Leu His Ala Lys Leu Ala Ser Glu Val Arg Ala Ala Ala Pro
                325                 330                 335

gcc ggc ggc ggc gag atc acc atc tcc gcc gtg gag aag gag atg ccg 1056
Ala Gly Gly Gly Glu Ile Thr Ile Ser Ala Val Glu Lys Glu Met Pro
            340                 345                 350

ctg gtg aag tcg gtg gtg tgg gag gcg ctg cgc atg aac ccg ccg gtg 1104
Leu Val Lys Ser Val Val Trp Glu Ala Leu Arg Met Asn Pro Pro Val
        355                 360                 365

gag ttc cag tac ggg cgc gcg cgg cgc gac atg gtc gtc gag agc cac 1152
Glu Phe Gln Tyr Gly Arg Ala Arg Arg Asp Met Val Val Glu Ser His
    370                 375                 380

gac gcg gcg tac gag gtc cgc aag ggg gag ctg ctg ttc ggg tac cag 1200
Asp Ala Ala Tyr Glu Val Arg Lys Gly Glu Leu Leu Phe Gly Tyr Gln
385                 390                 395                 400

ccg ctc gcc acc cgc gac gag aag gtg ttc gac cgc gcc ggc gag ttc 1248
Pro Leu Ala Thr Arg Asp Glu Lys Val Phe Asp Arg Ala Gly Glu Phe
                405                 410                 415

gtc ccc gac cgg ttc gtc tcc ggc gcc gga agc gcc gcc cgg ccg ctg 1296
Val Pro Asp Arg Phe Val Ser Gly Ala Gly Ser Ala Ala Arg Pro Leu
            420                 425                 430

ctg gag cac gtg gtg tgg tcg aac ggg ccg gag acc ggg acg cca tcg 1344
Leu Glu His Val Val Trp Ser Asn Gly Pro Glu Thr Gly Thr Pro Ser
        435                 440                 445

gag ggg aac aag cag tgc ccc ggg aag gac atg gtg gtg gcg gtg ggg 1392
Glu Gly Asn Lys Gln Cys Pro Gly Lys Asp Met Val Val Ala Val Gly
    450                 455                 460

cgg ctg atg gtg gcg ggg ctg ttc cgg cgg tac gac acg ttc gcc gcc 1440
Arg Leu Met Val Ala Gly Leu Phe Arg Arg Tyr Asp Thr Phe Ala Ala

465                 470                 475                 480

gac gtg gag gag ctg ccg ctt gag ccg gtg gtc acg ttc acg tcg ctg      1488
Asp Val Glu Glu Leu Pro Leu Glu Pro Val Val Thr Phe Thr Ser Leu
                485                 490                 495

acc cgc gcc gcc gac ggc gac ggc gcc gcg cgg cgc gga gta taa          1533
Thr Arg Ala Ala Asp Gly Asp Gly Ala Ala Arg Arg Gly Val
            500                 505                 510

<210>  2
<211>  510
<212>  PRT
<213>  Oryza sativa

<400>  2

Met Ala Pro Pro Arg Ala Asn Ser Gly Asp Gly Asn Asp Gly Ala Val
1               5                   10                  15

Gly Gly Gln Ser Lys Leu Ser Pro Ser Gly Leu Leu Ile Arg Glu Ile
            20                  25                  30

Pro Gly Gly Tyr Gly Val Pro Phe Leu Ser Pro Leu Arg Asp Arg Leu
        35                  40                  45

Asp Tyr Tyr Tyr Phe Gln Gly Ala Asp Glu Phe Phe Arg Ser Arg Val
    50                  55                  60

Ala Arg His Gly Gly Ala Thr Val Leu Arg Val Asn Met Pro Pro Gly
65                  70                  75                  80

Pro Phe Leu Ala Gly Asp Pro Arg Val Val Ala Leu Leu Asp Ala Arg
                85                  90                  95

Ser Phe Arg Val Leu Leu Asp Asp Ser Met Val Asp Lys Ala Asp Thr
            100                 105                 110

Leu Asp Gly Thr Phe Met Pro Ser Leu Ala Leu Phe Gly Gly His Arg
        115                 120                 125

Pro Leu Ala Phe Leu Asp Ala Ala Asp Pro Arg His Ala Lys Ile Lys
    130                 135                 140

Arg Val Val Met Ser Leu Ala Ala Ala Arg Met His His Val Ala Pro
145                 150                 155                 160

Ala Phe Arg Ala Ala Phe Ala Ala Met Phe Asp Glu Val Asp Ala Gly
                165                 170                 175

Leu Val Ala Gly Gly Pro Val Glu Phe Asn Lys Leu Asn Met Arg Tyr
            180                 185                 190

Met Leu Asp Phe Thr Cys Ala Ala Leu Phe Gly Gly Ala Pro Pro Ser
        195                 200                 205

Lys Ala Met Gly Asp Ala Ala Val Thr Lys Ala Val Lys Trp Leu Ile
    210                 215                 220

Phe Gln Leu His Pro Leu Ala Ser Lys Val Val Lys Pro Trp Pro Leu

225                 230                 235                 240

Glu Asp Leu Leu Leu His Thr Phe Arg Leu Pro Pro Phe Leu Val Arg
                245                 250                 255

Arg Glu Tyr Gly Glu Ile Thr Ala Tyr Phe Ala Ala Ala Ala Ala Ala
            260                 265                 270

Ile Leu Asp Asp Ala Glu Lys Asn His Pro Gly Ile Pro Arg Asp Glu
        275                 280                 285

Leu Leu His Asn Leu Val Phe Val Ala Val Phe Asn Ala Tyr Gly Gly
    290                 295                 300

Phe Lys Ile Phe Leu Pro His Ile Val Lys Trp Leu Ala Arg Ala Gly
305                 310                 315                 320

Pro Glu Leu His Ala Lys Leu Ala Ser Glu Val Arg Ala Ala Ala Pro
                325                 330                 335

Ala Gly Gly Gly Glu Ile Thr Ile Ser Ala Val Glu Lys Glu Met Pro
            340                 345                 350

Leu Val Lys Ser Val Val Trp Glu Ala Leu Arg Met Asn Pro Pro Val
        355                 360                 365

Glu Phe Gln Tyr Gly Arg Ala Arg Arg Asp Met Val Val Glu Ser His
    370                 375                 380

Asp Ala Ala Tyr Glu Val Arg Lys Gly Glu Leu Leu Phe Gly Tyr Gln
385                 390                 395                 400

Pro Leu Ala Thr Arg Asp Glu Lys Val Phe Asp Arg Ala Gly Glu Phe
                405                 410                 415

Val Pro Asp Arg Phe Val Ser Gly Ala Gly Ser Ala Ala Arg Pro Leu
            420                 425                 430

Leu Glu His Val Val Trp Ser Asn Gly Pro Glu Thr Gly Thr Pro Ser
        435                 440                 445

Glu Gly Asn Lys Gln Cys Pro Gly Lys Asp Met Val Val Ala Val Gly
    450                 455                 460

Arg Leu Met Val Ala Gly Leu Phe Arg Arg Tyr Asp Thr Phe Ala Ala
465                 470                 475                 480

Asp Val Glu Glu Leu Pro Leu Glu Pro Val Val Thr Phe Thr Ser Leu
                485                 490                 495

Thr Arg Ala Ala Asp Gly Asp Gly Ala Ala Arg Arg Gly Val
            500                 505                 510

<210>  3
<211>  30
<212>  DNA
<213>  Artificial

<220>
<223>  primer for AK105964

<400>  3
gcggatccat ggcgcgccgc cgcgagccaa                                        30


<210>  4
<211>  29
<212>  DNA
<213>  Artificial

<220>
<223>  primer for AK105964

<400>  4
gcgaattccc gcaccaacac tcgccgctc                                         29


<210>  5
<211>  1503
<212>  DNA
<213>  Oryza sativa

<220>
<221>  CDS
<222>  (1)..(1503)
<223>

<400>  5
atg gcg cca ccg cca gtg aac tcc ggc gac gcc gcc gcc gcc gcc acg      48
Met Ala Pro Pro Pro Val Asn Ser Gly Asp Ala Ala Ala Ala Ala Thr
1               5                   10                  15

gga gag aag agc aag ctc tcg ccg tcg ggc ctc ccc ata cgc gag ata      96
Gly Glu Lys Ser Lys Leu Ser Pro Ser Gly Leu Pro Ile Arg Glu Ile
            20                  25                  30

ccc ggc ggc tac ggc gtg ccc ttc ttc tcg ccg ctg cgc gac cgc ctc      144
Pro Gly Gly Tyr Gly Val Pro Phe Phe Ser Pro Leu Arg Asp Arg Leu
        35                  40                  45

gac tac ttc tac ttc cag ggc gcc gag gag tac ttc cga tca cgc gtc      192
Asp Tyr Phe Tyr Phe Gln Gly Ala Glu Glu Tyr Phe Arg Ser Arg Val
    50                  55                  60

gcc cgc cac ggc ggc gcc acc gtg ctc cgc gtc aac atg ccg ccc ggc      240
Ala Arg His Gly Gly Ala Thr Val Leu Arg Val Asn Met Pro Pro Gly
65                  70                  75                  80

ccc ttc atc tcc ggc aac ccc cgc gtc gtc gcc ctc ctc gac gcg cgc      288
Pro Phe Ile Ser Gly Asn Pro Arg Val Val Ala Leu Leu Asp Ala Arg
                85                  90                  95

agc ttc cgc gtc ctc ctc gac gac tcc atg gtg gac aag gcc gac acg      336
Ser Phe Arg Val Leu Leu Asp Asp Ser Met Val Asp Lys Ala Asp Thr
            100                 105                 110

ctc gac ggc acc tac atg ccg tcg cgc gcg ctc ttc ggc ggc cac cgc      384
Leu Asp Gly Thr Tyr Met Pro Ser Arg Ala Leu Phe Gly Gly His Arg
        115                 120                 125

ccg ctc gcc ttc ctc gac gcc gcc gac ccg cgc cac gcc aag atc aag      432
Pro Leu Ala Phe Leu Asp Ala Ala Asp Pro Arg His Ala Lys Ile Lys
    130                 135                 140

cgc gtc gtg atg tcg ctc gcc gcc gcg cgg atg cac cac gtc gcg ccg      480
Arg Val Val Met Ser Leu Ala Ala Ala Arg Met His His Val Ala Pro
145                 150                 155                 160

gcg ttc cgc gcc gcc ttt gcc gcc atg ttc gac gcc gtc gag gcc ggc      528
Ala Phe Arg Ala Ala Phe Ala Ala Met Phe Asp Ala Val Glu Ala Gly
                165                 170                 175

ctc ggc gcc gcc gtc gag ttc aac aag ctc aac atg agg tac atg ctc 576
Leu Gly Ala Ala Val Glu Phe Asn Lys Leu Asn Met Arg Tyr Met Leu
            180                 185                 190

gac ttc acc tgc gcc gcg ctg ttc ggc ggc gag ccg ccg agc aag gtg 624
Asp Phe Thr Cys Ala Ala Leu Phe Gly Gly Glu Pro Pro Ser Lys Val
        195                 200                 205

gtc ggc gac ggc gcc gtg acg aag gcc atg gcg tgg ctc gcg ttc cag 672
Val Gly Asp Gly Ala Val Thr Lys Ala Met Ala Trp Leu Ala Phe Gln
    210                 215                 220

ctg cac ccg atc gcg agc aag gtc gtc aag cca tgg ccg ctc gag gag 720
Leu His Pro Ile Ala Ser Lys Val Val Lys Pro Trp Pro Leu Glu Glu
225                 230                 235                 240

cta ctc ctg cac acc ttc tcc ctg ccg ccg ttc ctg gtg cgg cgt ggc 768
Leu Leu Leu His Thr Phe Ser Leu Pro Pro Phe Leu Val Arg Arg Gly
                245                 250                 255

tac gcc gac ctg aag gcg tac ttc gcc gac gcc gcc gcg gcc gtc ctc 816
Tyr Ala Asp Leu Lys Ala Tyr Phe Ala Asp Ala Ala Ala Ala Val Leu
            260                 265                 270

gac gac gcc gag aag agc cac acg gga atc ccg cgc gac gag ctc ctc 864
Asp Asp Ala Glu Lys Ser His Thr Gly Ile Pro Arg Asp Glu Leu Leu
        275                 280                 285

gac aac ctt gtg ttc gtc gcc att ttc aac gcc ttc ggc ggc ttc aag 912
Asp Asn Leu Val Phe Val Ala Ile Phe Asn Ala Phe Gly Gly Phe Lys
    290                 295                 300

atc ttc ctg cca cac atc gtc aag tgg ctc gcc cgc gcc ggc ccg gag 960
Ile Phe Leu Pro His Ile Val Lys Trp Leu Ala Arg Ala Gly Pro Glu
305                 310                 315                 320

ctc cac gcc aag ctt gcc acc gag gtc cgc gcc acc gtg ccc acc ggc 1008
Leu His Ala Lys Leu Ala Thr Glu Val Arg Ala Thr Val Pro Thr Gly
                325                 330                 335

gag gac gac ggc atc acc ctc gcc gcc gtc gag cgg atg ccg ctg gtg 1056
Glu Asp Asp Gly Ile Thr Leu Ala Ala Val Glu Arg Met Pro Leu Val
            340                 345                 350

aag tcg gtg gtg tgg gag gcg ctg cgc atg aac ccg ccg gtg gag ttc 1104
Lys Ser Val Val Trp Glu Ala Leu Arg Met Asn Pro Pro Val Glu Phe
        355                 360                 365

cag tac ggc cac gcg cgg cgc gac atg gtg gtc gag agc cac gac gcg 1152
Gln Tyr Gly His Ala Arg Arg Asp Met Val Val Glu Ser His Asp Ala
    370                 375                 380

gcg tac gag gtg cgc aag ggg gag atg ctg ttc ggc tac cag ccg ctc 1200
Ala Tyr Glu Val Arg Lys Gly Glu Met Leu Phe Gly Tyr Gln Pro Leu
385                 390                 395                 400

gcc acc cgc gac gag aag gtg ttc gac cgc gcc ggc gag ttc gtc gcc 1248
Ala Thr Arg Asp Glu Lys Val Phe Asp Arg Ala Gly Glu Phe Val Ala
                405                 410                 415

gac cgg ttc gtc gcc ggc ggc gcc gcc ggc gac cgg ccg ctg ctg gag 1296
Asp Arg Phe Val Ala Gly Gly Ala Ala Gly Asp Arg Pro Leu Leu Glu
            420                 425                 430

cac gtg gtg tgg tcg aac ggg ccg gag acg agg gcg cca tcg gag ggg 1344
His Val Val Trp Ser Asn Gly Pro Glu Thr Arg Ala Pro Ser Glu Gly
        435                 440                 445

aac aag cag tgc ccc ggg aag gac atg gtg gtg gcg gtg ggg cgg ctg 1392
Asn Lys Gln Cys Pro Gly Lys Asp Met Val Val Ala Val Gly Arg Leu
    450                 455                 460

atg gtg gcg gag ctg ttc cgg cgg tac gac acg ttc gcc gcc gac gtg 1440
Met Val Ala Glu Leu Phe Arg Arg Tyr Asp Thr Phe Ala Ala Asp Val
465                 470                 475                 480

gtg gag gcg ccg gtg gag ccg gtg gtg acg ttc acg tcg ctg aca cgg     1488
Val Glu Ala Pro Val Glu Pro Val Val Thr Phe Thr Ser Leu Thr Arg
                485                 490                 495

gcg tcg tcg gga tag                                                 1503
Ala Ser Ser Gly
            500

<210> 6
<211> 500
<212> PRT
<213> Oryza sativa

<400> 6

Met Ala Pro Pro Pro Val Asn Ser Gly Asp Ala Ala Ala Ala Ala Thr
1               5                   10                  15

Gly Glu Lys Ser Lys Leu Ser Pro Ser Gly Leu Pro Ile Arg Glu Ile
            20                  25                  30

Pro Gly Gly Tyr Gly Val Pro Phe Phe Ser Pro Leu Arg Asp Arg Leu
        35                  40                  45

Asp Tyr Phe Tyr Phe Gln Gly Ala Glu Glu Tyr Phe Arg Ser Arg Val
    50                  55                  60

Ala Arg His Gly Gly Ala Thr Val Leu Arg Val Asn Met Pro Pro Gly
65                  70                  75                  80

Pro Phe Ile Ser Gly Asn Pro Arg Val Val Ala Leu Leu Asp Ala Arg
                85                  90                  95

Ser Phe Arg Val Leu Leu Asp Asp Ser Met Val Asp Lys Ala Asp Thr
            100                 105                 110

Leu Asp Gly Thr Tyr Met Pro Ser Arg Ala Leu Phe Gly Gly His Arg
        115                 120                 125

Pro Leu Ala Phe Leu Asp Ala Ala Asp Pro Arg His Ala Lys Ile Lys
    130                 135                 140

Arg Val Val Met Ser Leu Ala Ala Ala Arg Met His His Val Ala Pro
145                 150                 155                 160

Ala Phe Arg Ala Ala Phe Ala Ala Met Phe Asp Ala Val Glu Ala Gly
                165                 170                 175

Leu Gly Ala Ala Val Glu Phe Asn Lys Leu Asn Met Arg Tyr Met Leu
            180                 185                 190

Asp Phe Thr Cys Ala Ala Leu Phe Gly Gly Glu Pro Pro Ser Lys Val
        195                 200                 205

Val Gly Asp Gly Ala Val Thr Lys Ala Met Ala Trp Leu Ala Phe Gln
    210                 215                 220

Leu His Pro Ile Ala Ser Lys Val Val Lys Pro Trp Pro Leu Glu Glu
225                 230                 235                 240

Leu Leu Leu His Thr Phe Ser Leu Pro Pro Phe Leu Val Arg Arg Gly
                245                 250                 255

Tyr Ala Asp Leu Lys Ala Tyr Phe Ala Asp Ala Ala Ala Ala Val Leu
            260                 265                 270

Asp Asp Ala Glu Lys Ser His Thr Gly Ile Pro Arg Asp Glu Leu Leu
        275                 280                 285

Asp Asn Leu Val Phe Val Ala Ile Phe Asn Ala Phe Gly Gly Phe Lys
    290                 295                 300

Ile Phe Leu Pro His Ile Val Lys Trp Leu Ala Arg Ala Gly Pro Glu
305                 310                 315                 320

Leu His Ala Lys Leu Ala Thr Glu Val Arg Ala Thr Val Pro Thr Gly
                325                 330                 335

Glu Asp Asp Gly Ile Thr Leu Ala Ala Val Glu Arg Met Pro Leu Val
            340                 345                 350

Lys Ser Val Val Trp Glu Ala Leu Arg Met Asn Pro Pro Val Glu Phe
        355                 360                 365

Gln Tyr Gly His Ala Arg Arg Asp Met Val Val Glu Ser His Asp Ala
    370                 375                 380

Ala Tyr Glu Val Arg Lys Gly Glu Met Leu Phe Gly Tyr Gln Pro Leu
385                 390                 395                 400

Ala Thr Arg Asp Glu Lys Val Phe Asp Arg Ala Gly Glu Phe Val Ala
                405                 410                 415

Asp Arg Phe Val Ala Gly Gly Ala Ala Gly Asp Arg Pro Leu Leu Glu
            420                 425                 430

His Val Val Trp Ser Asn Gly Pro Glu Thr Arg Ala Pro Ser Glu Gly
        435                 440                 445

Asn Lys Gln Cys Pro Gly Lys Asp Met Val Val Ala Val Gly Arg Leu
    450                 455                 460

Met Val Ala Glu Leu Phe Arg Arg Tyr Asp Thr Phe Ala Ala Asp Val
465                 470                 475                 480

Val Glu Ala Pro Val Glu Pro Val Val Thr Phe Thr Ser Leu Thr Arg
                485                 490                 495

Ala Ser Ser Gly
            500

<210>  7
<211>  26
<212>  DNA
<213>  Artificial

<220>
<223>  primer for AK107161

<400>  7
gccggatcca tggcgccacc gccagt                                          26


<210>  8
<211>  33
<212>  DNA
<213>  Artificial

<220>
<223>  primer for AK107161

<400>  8
gccgaattcc gcaattaaaa catccaccaa cct                                  33

## Description

### Technical Field

The present invention relates to 9-fatty acid hydroperoxide lyase genes.

### Background Art

Cereals used as food and food raw materials contain lipids and fatty acids. When the cereals are processed or preserved, these lipids and fatty acids are oxidized by autoxidation due to the lipoxygenase contained in the cereals, or by oxygen in the air, and produce lipid hydroperoxides and fatty acid hydroperoxides. These hydroperoxides then produce aldehydes by pyrolysis, chemical degradation and enzymatic degradation, and since the produced aldehydes have a disagreeable odor or fatty acid odor, the flavor of the cereals is considerably impaired thereby (Non-patent Document 1).

Among these aldehydes, *trans-2-nonenal* is known to be an aldehyde with a low odor threshold, and is thought to be responsible for the disagreeable odor of old beer which has been kept for a long time (Non-patent Document 2), or rice which has been kept for a long time that has suffered quality deterioration due to processing or storage (Non-patent Document 3 or 4).

The production of *trans-2-nonenal* in beer may for example occur according to the following mechanism (Non-patent Document 5). In beer brewing, there is a brewing process which saccharifies malt to yield wort. In this process, linoleic acid 9-hydroperoxide, a kind of fatty acid hydroperoxide, is produced. The produced linoleic acid 9-hydroperoxide undergoes cleavage by the 9-fatty acid hydroperoxide lyase in the beer raw materials such as malt, thereby producing *trans-2-nonenal.*

Aldehyde synthesis in plants by oxylipin synthesis system has been studied in detail for dicotyledons (Non-patent Document 6). Aldehydes are produced by a cascade reaction between lipoxygenase and fatty acid hydroperoxide lyase, and it is thought that the fatty acid hydroperoxide lyase is an enzyme which catalyzes the rate-determining step of this reaction system. It has been determined that among these various aldehydes, *trans-2-nonenal* is produced by catalysis of a cleavage reaction of linoleic acid 9-hydroperoxide by 9-fatty acid hydroperoxide lyase (Non-patent Document 7), and the structure of the linoleic acid 9-hydroperoxide lyase gene has also been clarified (Non-patent Document 8).

There has been little research on monocotyledons such as rice and wheat, and a fatty acid hydroperoxide lyase gene have been isolated only in barley. However, this fatty acid hydroperoxide lyase cannot use linoleic acid 9-hydroperoxide as a substrate, and therefore it does not participate in the production of *trans-2-nonenal* at all (Non-patent Document 9).

[Non-patent Document 1]: J. Bruce German, Food Processing and Lipid Oxidation, Impact of Processing on Food Safety Kluwer Academic/Plenum Publishers, New York, 1999.
[Non-patent Document 2]: Drost, B.W. et al, Flavor stability, J. Am. Soc. Brew. Chem., 48, 124-131. 1990.
[Non-patent Document 3]: Suzuki et al, Volatile components in stored rice (Oryza sativa (L.)) of varieties with and without lipoxygenase-3 in seeds, J. Agric. Food Chem., 47: 1119, 1999.
[Non-patent Document 4]: Lam. H.S. and Proctor A., Milled Rice Oxidation Volatiles and Odor Development, Journal of Food Science, 68: 2676-2681,2003.
[Non-patent Document 5]: Kuroda et al, Characterization of factors involved in the production of 2(E)-nonenal during mashing, Biosci. Biotechnol. Biochem., 67: 691-697, 2003.
[Non-patent Document 6]: Blee, E., Phytooxylipins and plant defense reactions, Prog. Lipid Res., 37, 33-72, 1998.
[Non-patent Document 7]: Matsui et al, Fatty acid 9-and 13-hydroperoxide lyases from cucumber, FEBS Lett. 481, 183-8, 2000.
[Non-patent Document 8]: Matsui, K. et al, Bell pepper fruit fatty acid hydroperoxide lyase is a cytochrome P450 (CYP74B) FEBS Lett., 394, 21-24, 1996.
[Non-patent Document 9]: Koeduka et al, Kinetics of barley FA hydroperoxide lyase are modulated by salts and detergents, Lipids, 38: 1167-1172,2003.

### Disclosure of the Invention

### Problems to be Solved by the Invention

If the 9-fatty acid hydroperoxide lyase gene of monocotyledons were elucidated, a restriction fragment length polymorphism (RFLP) and single nucleotide polymorphism (SNP) could then be found, and it would be possible to select a strain from which 9-fatty acid hydroperoxide lyase had been eliminated, or a strain wherein the stability and activity of 9-fatty acid hydroperoxide lyase was low. It would also be possible to produce a strain in which 9-fatty acid hydroperoxide lyase activity was suppressed by a gene suppressing technique such as RNA interference (RNAi).

It is therefore an object of the invention to elucidate a 9-fatty acid hydroperoxide lyase gene of monocotyledons.

### Means for Solving the Problems

In recent years, although a large amount of genetic information has been collected by genome techniques, and it is possible to extract similar genes based on existing genetic information, it is very difficult to identify a catalytic action or a product. Although fatty acid hydroperoxide lyase belongs to the cytochrome P450 family, this family consists of thousands of kinds of similar gene clusters, and it is impossible to estimate the function of an enzyme protein from the similarity of gene sequences.

The Inventors screened many genes similar to fatty acid hydroperoxide lyase based on published genetic information for rice, and as a result of expressing these genes and investigating their functions, discovered that a gene consisting of the nucleotide sequence as set forth in SEQ ID No: 1 and a gene consisting of the nucleotide sequence as set forth in SEQ ID No: 5 converted linoleic acid 9-hydroperoxide into *trans-2-nonenal,* and thus arrived at the present invention.

Specifically, the invention provides a 9-fatty acid hydroperoxide lyase gene consisting of the nucleotide sequence as set forth in SEQ ID No: 1. The invention further provides a 9-fatty acid hydroperoxide lyase gene which encodes 9-fatty acid hydroperoxide lyase consisting of the amino acid sequence as set forth in SEQ ID No: 2.

The invention further provides a 9-fatty acid hydroperoxide lyase gene consisting of the nucleotide sequence as set forth in SEQ ID No: 5. The invention also provides a 9-fatty acid hydroperoxide lyase gene which encodes 9-fatty acid hydroperoxide lyase consisting of the amino acid sequence as set forth in SEQ ID No: 6.

### Effect of the Invention

Taking the gene of the invention as an indicator, it is possible to select a strain of 9-fatty acid hydroperoxide lyase with low stability and activity. By controlling or modifying the expression of the gene of the invention, it is also possible to produce a strain of 9-fatty acid hydroperoxide lyase with low stability and activity. This means that food can be provided which keeps its flavor over time.

### Brief Description of the Drawings

FIG. 1 is a phylogenetic tree relating to AK105964 and AK107161.
FIG. 2(a) is a total ion chromatogram of GC-MS of a reaction product of a protein obtained by expressing the gene consisting of the nucleotide sequence as set forth in SEQ ID No: 1, with linoleic acid 9-hydroperoxide. (b) is a total ion chromatogram of a negative control.
FIG. 3 is a mass spectrogram of peak 1.
FIG. 4 is a graph showing *trans-2-nonenal* produced by reaction of a protein obtained by expressing the nucleotide sequence as set forth in SEQ ID No: 1, with linoleic acid 9-hydroperoxide.
FIG. 5(a), (b) and (c) are total ion chromatograms of GC-MS of reaction products of 13-HPOD, 9-HPOT and 13-HPOT and a protein obtained by expressing the gene consisting of the nucleotide sequence as set forth in SEQ ID No: 1, respectively.

### Description of Reference Numerals

1*- trans-2-nonenal,* 2 - 1-nonanol (internal standard).

### Best Mode for Carrying Out the Invention

One of the 9-fatty acid hydroperoxide lyase genes of the invention derived from rice consists of the nucleotide sequence as set forth in SEQ ID No: 1 of the Sequence Listing. The 9-fatty acid hydroperoxide lyase polypeptide encoded by this gene consists of the amino acid sequence as set forth in SEQ ID No: 2 of the Sequence Listing. Another 9-fatty acid hydroperoxide lyase gene of the invention derived from rice consists of the nucleotide sequence as set forth in SEQ ID No: 5 of the Sequence Listing. The 9-fatty acid hydroperoxide lyase polypeptide encoded by this gene consists of the amino acid sequence as set forth in SEQ ID No: 6 of the Sequence Listing. 9-fatty acid hydroperoxide lyase converts linoleic acid 9-hydroperoxide into *trans-2-nonenal* which is responsible for the aging odor. Therefore, by suppressing the expression of, or converting the gene of the invention, the flavor of cereals can be maintained, and the gene of the invention can be used for such a purpose.

For example, there is a method of using RNAi and antisense RNA to control the expression of the gene of the invention and maintain the flavor of cereals. It is also possible to produce cereals which maintain their flavor by producing a 9-fatty acid hydroperoxide lyase gene with decreased *trans-2-nonenal* production activity using site-specific mutagenesis etc., and introducing this gene into cereals. As the introduction method, the Agrobacterium method, protoplast method, PEG method, electroporation method, particle gun method or microinjection method may be used.

The gene of the invention can be obtained from rice (for example, strains such as Nipponbare) expressing this gene, using RT-PCR method. More specifically, the gene of the invention can be obtained by extracting RNA from the rice by AGPC method, or guanidine/cesium chloride ultracentrifugation method, etc., and performing PCR using the obtained RNA as a template.

A gene which encodes 9-fatty acid hydroperoxide lyase which is a mutant of the gene of the invention, can also be synthesized by site-specific mutagenesis. As a method of introducing a mutation into a nucleic acid, a known method such as Kunkel method or Gapped duplex method, may be mentioned. For example, the mutation can be introduced using a mutation introduction kit (Mutan-K or Mutan-G, TAKARA), or a LA PCR in vitro Mutagenesis series kit (TAKARA).

### Examples

### (Example 1)

Using barley 13-fatty acid hydroperoxide lyase (CAC82980), barley rice allene oxide synthetase (CAB86383, CAB86384) and rice allene oxide synthetase (Q8VZX5, Q940D7), which are functionally known genes, homologous proteins were extracted using the protein-protein BLAST program in the database of NCBI (National Center for Biotechnology Information) and KOME (Knowledge-Based Oryza Molecular Biological Encyclopedia), setting an E-value = e⁻¹⁰ as a threshold.

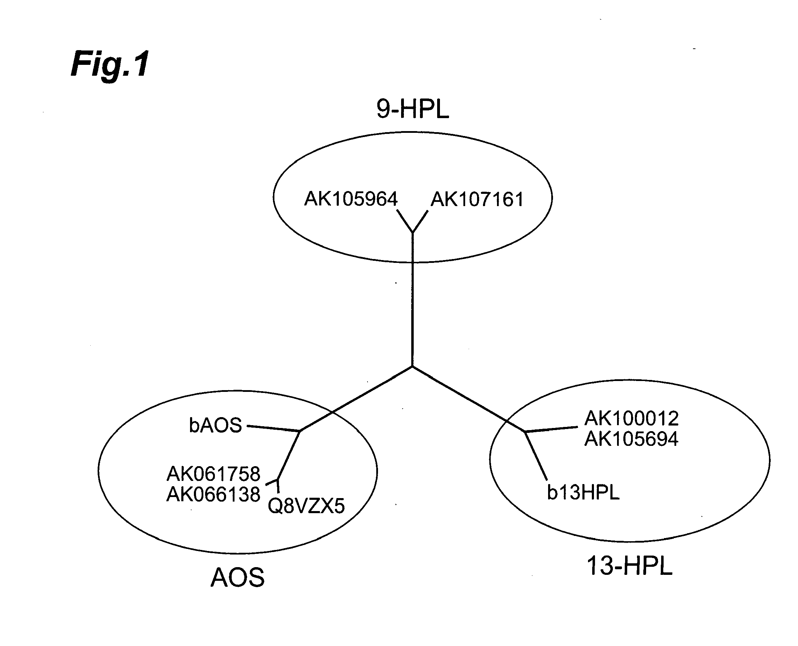

As a result, rice cDNAs AK105964 and AK107161 were newly extracted. When a molecular genealogical tree was created on an ExPASy Molecular Biology Server using ClastaIW, the molecular genealogical tree shown in FIG. 1 was obtained. As shown in FIG. 1, the two newly extracted genes were classified into clearly different groups from the allene oxide synthetase gene (AOS) and 13-fatty acid hydroperoxide lyase (13-HPL), and it was expected that they would have different enzyme functions from those of AOS and 13-HPL. Since AK105964 and AK107161 had very similar structures, a functional analysis was undertaken for AK105964.

### (Example 2)

After extraction of total RNA from rice (strain: Nipponbare) using a Tripure Isolation Reagent (Roche), PolyA⁺ RNA was purified using an Oligotex-dT30 mRNA Purification Kit (TAKARA Bio). RT-PCR was performed using two primers (5'-gcggatccatggcgcgccgccgcgagccaa-3' (SEQ ID No: 3) and 5'-gcgaattcccgcaccaacactcgccgctc-3' (SEQ ID No: 4)), and subcloning into pUC118 was performed. The full length sequencing was performed, and it was confirmed that the sequence matched AK105964. Next, the structural gene of AK105964 was subcloned between the BamHI site and EcoRI site of pRSETA (Invitrogen), and introduced into the *E. coli* strain BL21(DE3)pLys.

For expression of *E. coli* and preparation of a crude extract, the method disclosed in Kuroda et al., Characterization of factors that transform linoleic acid into di- and trihydroxyoctadecenoic acids in mash, J. Biosci. Bioeng., 93: 73-77, 2002, was used. Specifically, isopropyl-β-thiogalactopyranoside was added to 50ml of culture medium (OD600=0.5) pre-cultured at 16°C so that the final concentration was 1mM. Culture was continued for further 16 hours, *E. coli* cells were centrifuged and recovered, and suspended by adding 5mL of Tris-HCl buffer solution (pH 7.2) containing 0.1% of Tween 20. After lysing the cells by sonication of the cell suspension and centrifuging for 30 minutes (15,000g, 4°C), the supernatant liquid was collected and taken as a crude extract. As a control sample, BL21(DE3)pLys into which pRSETA had been introduced was prepared, and a crude extract was obtained under the same conditions as those described above.

The activity measurement of fatty acid hydroperoxide lyase was performed according to the method disclosed in Kuroda et al., Characterization of factors involved in the production of 2(E)-noneal during mashing, Biosci. Biotechnol. Biochem., 67: 691-697, 2003. 10mM Tris-HCl buffer solution (pH 7.2) containing 0.1% of Tween 20 was added to 5µL of the crude extract and made up to 100µL, then 5µL of linoleic acid 9-hydroperoxide (2mM) dissolved in ethanol was added, incubated for 5 minutes at 30°C, and the reaction was stopped by adding 900µL of ethanol. The reactants were transferred to a vial to which 3g of sodium chloride and 5mL of distilled water had been added, 1 ppm of 1-nonanol (ethanol solution) was added as an internal standard, and the vial was capped.

Dosage and identification of compounds by gas chromatography-mass spectrometry (GC-MS) were performed as follows. A polydimethyl siloxane SPME fiber (Spelco) was inserted in the vial, and after incubating at 40°C for 10 minutes, it was supplied to an injection port (260°C) of a gas chromatography (Hewlett Packard HP6890/MSD system). The capillary column was DB-1 (30m x 0.25mm, film thickness 1µm, J&W Inc.), and the carrier gas was helium (1mL/min). The oven conditions were 120°C maintained for 1 minute, temperature increase up to 150°C at 5°C/min, temperature increase up to 300°C at 40°C/min, and temperature maintained at 300°C for 2 minutes. The dosages of *trans-2-nonenal* and 1-nonanol were analyzed in the selected ion mode (m/z). The retention times of *trans-2-nonenal* and 1-nonanol were 7.6 minutes and 7.8 minutes, respectively. For GC-MS analysis, gas chromatography was performed as described above, and MS/MS analysis of the product and compound identification were performed using HP-Chemistation software and compound library.

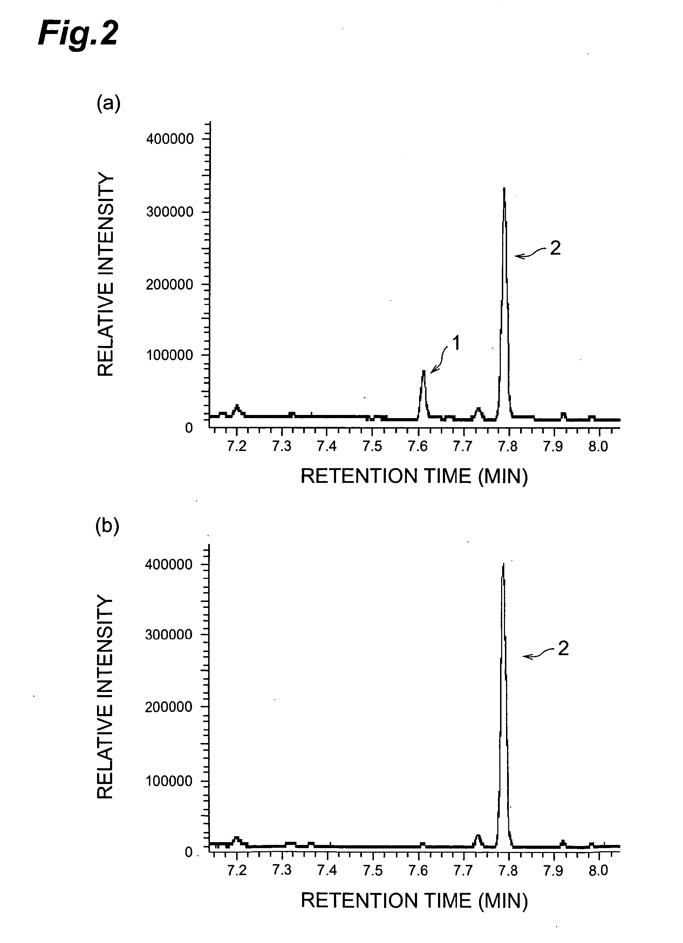

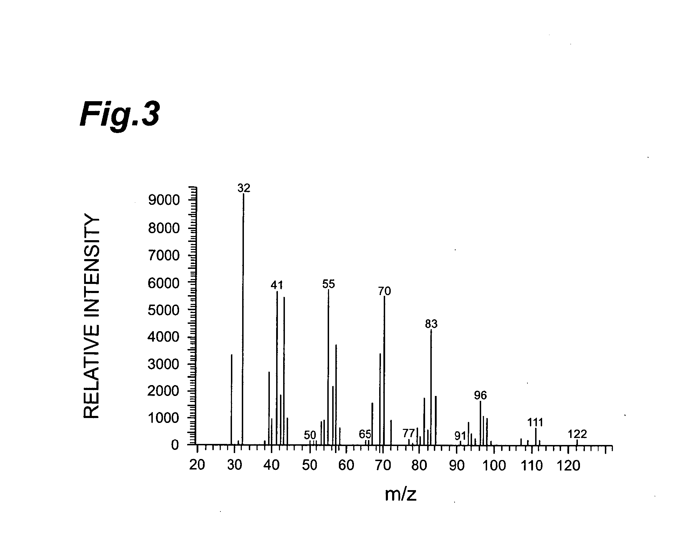

FIG. 2(a) is a diagram (total ion chromatogram) showing the GC-MS result for the reaction product of a protein obtained by expressing the gene consisting of the nucleotide sequence as set forth in SEQ ID No: 1 and linoleic acid 9-hydroperoxide. FIG. 2(b) shows a total ion chromatogram of a negative control. Peak 1 at a retention time of 7.6 minutes represents *trans-2-nonenal,* and peak 2 at a retention time of 7.8 minutes represents 1-nonanol, the internal standard. As shown in FIG. 2, the peak of *trans-2-nonenal* was observed only when the gene consisting of the nucleotide sequence as set forth in SEQ ID No: 1 was expressed. FIG. 3 shows the mass spectrogram of peak 1, and from this mass spectrogram, it is seen that peak 1 represents *trans-2-nonenal.*

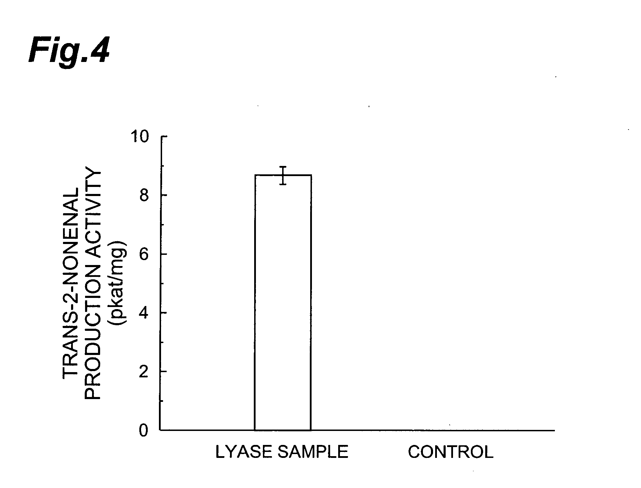

FIG. 4 is a graph showing *trans-2-nonenal* produced by reacting the protein obtained by expressing the gene consisting of the nucleotide sequence as set forth in SEQ ID No: 1 with linoleic acid 9-hydroperoxide. The vertical axis shows the activity of *trans-2-nonenal* per *E. coli* protein (mg). It is seen that *trans-2-nonenal* is produced only when the gene consisting of the nucleotide sequence as set forth in SEQ ID No: 1, is expressed.

An analysis was performed for AK107161 in an identical manner to that of AK105964. Using 5'-gccggatccatggcgccaccgccagt-3' (SEQ ID No: 7) and 5'-gccgaattccgcaattaaaacatccaccaacct-3' (SEQ ID No: 8) as primers, subcloning into pUC118 was performed. The full length sequencing was performed, and it was confirmed that the sequence matched AK107161. Next, the structural gene of AK107161 was subcloned between the BamHI site and EcoRI site of pRSETA, and introduced into *E. coli* strain BL21(DE3)pLys. Thereafter, it was expressed in *E. coli* by an identical method to that for AK105964, a crude extract was prepared, and it was found that AK107161 had an identical activity to that of AK105964.

From the above results, it was clear that the gene consisting of the nucleotide sequence as set forth in SEQ ID No: 1 and the gene consisting of the nucleotide sequence as set forth in SEQ ID No: 5, were 9-fatty acid hydroperoxide lyase genes which convert linoleic acid 9-hydroperoxide into *trans-2-nonenal.*

### (Example 3)

Next, the catalytic activities of the following fatty acid peroxides of AK105964 (and AK107161) (all peroxides were contained in rice), were analyzed: 13(S)-hydroperoxy-9(Z),11(E)-octadecadienoic acid (13-HPOD), 9(S)-hydroperoxy-10(E), 12(Z), 15(Z)-octadecatrienoic acid (9-HPOT) and 13(S)-hydroperoxy-9(Z),II(E),15(Z)-octadecatrienoic acid (13-HPOT). As in Example 2, an enzyme extract and the fatty acid peroxide were reacted together, and the obtained product was analyzed by GC-MS.

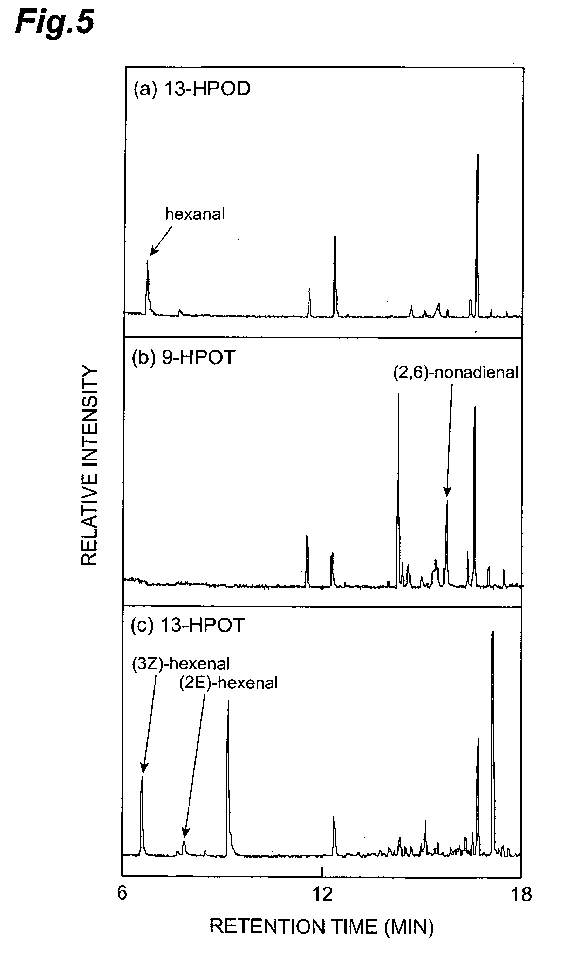

The obtained chromatograms are shown in FIG. 5. (a), (b) and (c) are GC-MS chromatograms of the products obtained by reacting with 13-HPOD, 9-HPOT and 13-HPOT, respectively. As shown in FIG. 5(a)-(c), hexanal was produced from 13-HPOD, (2,6)-nonadienal was produced from 9-HPOT, and (3Z)-hexenal and (2E)-hexenal was produced from 13-HPOT. The obtained aldehydes may be responsible for the off-flavor of foods, and it can be expected that the quality of rice will be improved by suppressing the expression of AK105964 (and AK107161) with gene suppressing techniques.

## Claims

1. A 9-fatty acid hydroperoxide lyase gene consisting of the nucleotide sequence as set forth in SEQ ID No: 1.

2. A fatty acid hydroperoxide lyase gene encoding a 9-fatty acid hydroperoxide lyase consisting of the amino acid sequence as set forth in SEQ ID No: 2.

3. A 9-fatty acid hydroperoxide lyase gene comprising the nucleotide sequence as set forth in SEQ ID No: 5.

4. A fatty acid hydroperoxide lyase gene encoding a 9-fatty acid hydroperoxide lyase consisting of the amino acid sequence as set forth in SEQ ID No: 6.
